# EUROPEAN PATENT SPECIFICATION

(11) **EP 0 760 676 B1**
(45) Date of publication and mention of the grant of the patent: **11.07.2001**
(21) Application number: 95921438.8
(22) Date of filing: 25.05.1995
(51) Int. Cl.: A61K 38/17, A61K 39/145

(54) **MATERIALS AND METHODS FOR TREATMENT OF PLAQUING DISEASES**
MITTEL UND METHODEN ZUR BEHANDLUNG VON PLAQUE-KRANKHEITEN
COMPOSITIONS ET PROCEDES DE TRAITEMENT DE MALADIES A FORMATION DE PLAQUE

(30) Priority: 25.05.1994 US 249175
(43) Date of publication of application: 12.03.1997
(73) Proprietor: McMICHAEL, John, Delanson, NY 12053 (US)
(72) Inventor: KLINE, Ellis, L., Pendleton, SC 29670 (US); McMICHAEL, John Corner of, Delanson, NY 12053 (US)
(74) Representative: Brown, John David
(86) International application number: US9506689
(87) International publication number: WO9531996

(56) References cited:
- WO-A-91/16819
- SCIENCE, vol. 243, 17 March 1989 LANCASTER, PA US, pages 1488-1490, J.S. WHITSON ET AL. 'AMYLOID BETA PROTEIN ENHANCES THE SURVIVAL OF HIPPOCAMPAL NEURONS IN VITRO.'

## Description

The present invention relates to materials for the treatment of diseases involving plaque formation.

Cellular skeletal systems have three distinct ultrastructural features, microtubules, intermediate filaments, and microfilaments, all of which are fibrous macromolecules associated with the central nervous system (CNS). Neuronal intermediate filaments, defined as neurofilaments (containing amyloid beta protein constructs), are distinct from other intermediate filaments found in the cells of the central nervous system. R.D. Goldman, A. Milstead, J.A. Schloss and M.J. Yerna, *Annu. Rev*. *Physiol*., 41, p. 703-722 (1979); R.J. Lasak, *Neurosci*. *Res*. *Program Bull.,* 19, p. 7-32 (1981); R.J. Lasek and M.L. Shelanski, *Neurosci*. *Res. Program Bull.,* 19, p. 3-153 (1981); C.A. Maretta, ed., *Neurofilaments* (1983); M.L. Shelanski and R.K.H. Liem, *J. Neurochem*., 33, *p.* 5-13 (1979). Neurofilaments are composed of three proteins with molecular weights of 200,000, 150,000 and 70,000 daltons. B.H. Toh, L.J. Gibbs, Jr., D.C. Gajdusek, J. Goudsmit and D. Dahl, *Proc*. *Natl*. *Acad*. *Sci*. *USA*. An additional 62,000 dalton protein is also affiliated with the abovementioned proteins. Such proteins are associated with slow axoplasmic transport. P.N. Hoffman and R.J. Lasek, *J*. *Cell Biology*, 66, p. 351-366 (1975).

Alzheimer's Disease, and other amyloid associated maladies including senile dementia, Down's syndrome, Pick's disease, progressive supranuclear palsy, multiple sclerosis and others, are characterized by the presence of one or more fused fibrils of repetitive amyloid beta proteins or other similar amyloid residues such as paired helical filaments, neurofibrillary tangles, neuritic plaques, amyloid plaques and cerebrovascular amyloidosis. B.H. Anderson, D. Breinberg and M.J. Downes, *Nature,* 298, p. 84-86 (1982). These paired helical filaments are indistinguishable immunologically and chemically from normal neurofilaments and share many of the same proteinaceous epitopes. B.H. Anderson, D. Breinberg and M.J. Downes, *Nature,* 298, p. 84-86 (1982); B.H. Toh, L.J. Gibbs, D.C. Gajdusek, J. Goudsmit and D. Dahl, *Proc. Natl*. *Acad*. *Sci*. *USA*; *K.* Iqbal, I. Grundke-Iqbal, H.M. Wisnieski and R.D. Terry, *Brain Res*., 142, p. 321-332 (1975). It has been suggested that they interfere with axonal transport. P.N. Hoffman and R.J. Lasek, *J*. *Cell*. *Biol*., 66, p. 351-366 (1975); J.W. Griffin, P.N. Hoffman, A.W. Clark, P.T. Carroll and D.L. Price, *Science,* 202, p. 633-665 (1978).

Using a cDNA clone of the gene encoding amyloid beta protein as a genetic probe, it was shown that the gene is located on chromosome twenty-one and is expressed in many tissues of the body. D. Goldjaber, M.I. Lerman, O.W. McBridge, U. Suffiotti and D.C. Gaidusak, *Science,* 235, p. 77-780 (1987); R.E. Tanzi, J.F. Gusella, P.C. Watkins, G.A.P. Bruns, P. St.George, M.L. Vankeuren, D. Patterson, S. Pagan, D.M. Kurnit and R.L. Neve, *Science,* 235, p. 880-884 (1987). Quantitation of amyloid beta protein expression, as seen by its mRNA levels using the cDNA probe, has revealed that its level of expression in brain tissue of Alzheimer's patients was not above that seen for other tissues outside the central nervous system. Such a finding was of interest to researchers when noting that amyloid plaque formation only occurs in the brain. R.E. Tanzi, J.F. Gusella, P.C. Watkins, G.A.P. Bruns, P. St.George, M.L. Vankeuren, D. Patterson, S. Pagan, D.M. Kurnit and R.L. Neve, *Science*, 235, p. 880-884 (1987).

Amyloid beta protein is obtained through conventional means known in the art and has been characterized in various reports. A.S. Cohen and E. Calkins, *Nature*, 183, p. 1202 (1959); A.S. Cohen and E. Calkins, *J*. *Cell Biology,* 21, p. 481 (1964); A.S. Cohen, E. Calkins and C. Levens, *Am*. *J*. *Pathol*., 35, *p. 979* (1959). More recent work is manifested by D. Caspi, M.C. Baltz and M.K. Pepys, *Mol*. *Biol*. *Med*., 3, pp. 387-407 (1986); and D. Caspi, M.C. Baltz and M.K. Pepys, *Mol*. *Biol*. *Med*., 3, pp. 409-424 (1986). Amyloid beta protein exists in various structural forms. The amyloid beta protein that has been experimentally used and as referred to herein in terms of any specific embodiments constitutes a mixture of such forms. It is to be understood that within the scope of the present invention, it is contemplated that any of the various forms of amyloid beta protein may be used.

Amyloid beta protein from the brain has been cDNA cloned and shown to contain a unique twenty amino acid NH₂-terminal sequence. Glenner, G.G. and Wong, W., *Biochem*. *Biophys*. *Res*. *Comm.,* 122, No. 3, pp. 1131-35 (1984); D. Caspi, M.C. Baltz and M.K. Pepys, *Mol*. *Biol. Med.,* 3, pp. 409-424 (1986); Goldgaber, D., Lerman, M.I., McBridge, O.W., Saffiotti, U. and Gaidusak, D.C., *Science,* 235, pp. 777-80 (1987).

It has been observed that a buildup of abnormally organized amyloid beta protein in brain tissue is manifested in Alzheimer's Disease. See Dennis J. Selkoe and Carmela R. Abraham, "Isolation of Paired Helical Filaments and Amyloid Fibers from Human Brain," 134, *Methods in Immunology,* 388-404 (1986). The fact that there is an accumulation of beta amyloid protein in the brain in Alzheimer patients has been demonstrated by post mortem analysis of brain tissue that manifest a concentration of amyloid beta protein as part of an accumulation of parallel filaments or neural fibrillatory tangles in the brain that appear characteristic of Alzheimer victims, along with neuritic plaque and cerebral vasculatory amyloidosis.

The presence of amyloid beta protein in fibrils and plaques in Alzheimer's Disease, as well as other CNS disorders, has been suggested to be a result of a degradation product of the normal neurofilaments, D. Goldjaber, M.I. Lerman, O.W. McBridge, U. Suffiotti and D.C. Gaidusak, *Science,* 235, p. 77-780 (1987); R.E. Tanzi, J.F. Gusella, P.C. Watkins, G.A.P. Bruns, P. St.George, M.L. Vankeuren, D. Patterson, S. Pagan, D.M. Kurnit and R.L. Neve, *Science,* 235, p. 880-884 (1987); M. Baudry, B.R. Dubrin, L. Beasley, M. Leon and G. Lynch, *Neurobiol*. *Aging*, 7, *p. 255-260* (1986); G.G. Glenner, *Arch. Path*. *Lab. Med*., 107, p. 218-282 (1983); or possibly due to improper metabolism of byproducts. Further breakdown products of amyloid beta proteins from neurofilaments have also been observed in amyloid plaques along meningeal vascular walls and intracortical blood vessels. S. Bahmanyar, E.J. Williams, F.B. Johnson, S. Young and D.C. Gaidusak, *J*. *Comp*. *Path,* 95, p. 1-5 (1985); M.E. Bruce and H. Fraser, *Neuropathol*. *Appl*. *Neurobiol*, 1, p. 189-207 (1981); M.E. Bruce and H. Fraser, *Neurophathol*. *Appl*. *Neurobiol*., 7, p. 289-298 (1981); G.G. Glenner and W. Wong, J. Quaranta and G.G. Glenner, *Proc*. *Natl*. *Acad*. *Sci*., 82, p. 8729 (1985); D.J. Selkoe, C.R. Abraham, M.B. Podlisky and L.K. Duffy, *J*. *Neurochem*., *46,* p. 1820 (1986).

During the mid-1960's, Solomon & Moos speculated that there was a close integration between immunological function, the central nervous system, psychophysiological factors (emotions), and disease, both physical and mental. G.F. Solomon and R.H. Moos, *Arch*. *Gen. Psychiatry*, 11, p. 657-674 (1964). The integration of those systems was initially suggested through observation of the presence of abnormal immunoglobulins in schizophrenic patients. G.F. Solomon and R.H. Moos, *Arch*. *Gen*. *Psychiatry*, 11, p. 657-674 (1964); J.G. Knight, *Lancet,* 82, p. 1073-1076 (1982); W.J. Fessel and M. Hirata-Hibi, *Arch. Gen. Psychiatry,* 9, p. 601-613. These immune aberrations (termed autoantibodies), which seemed to target certain body cellular structures, G.F. Solomon, *Psychoneuroimmunology*, *p. 259-278* (1985); G.F. Solomon and R.H. Moos, *Psychosom. Mod.,* 27, p. 135-149 (1981), supported the concept that there is a close communication between the CNS and the immune system. For instance, met-enkephalin is a neurotransmitter in the CNS and is a product of activated T-helper cells. G. Zurawaki, M. Benedik, D.J. Kamb, J.S. Abrams, S.M. Zurawaki and F.O. Lee, *Science,* 232, p. 772-775 (1986).

The appearance of autoantibodies specific to the CNS neurofilaments in patients with Alzheimer's and other CNS disorders suggests that the body's immune system may play a role in the disease process. S. Bahmanyar, R.K.H. Liem, J.W. Griffin and D.C. Gajdusek, *J. Neuropathol*. *Exp*. *Neurol*., 53, p. 85-90 (1984); S. Bahmanyar, M.C. Moreau-Dubois, P. Brown, F. Catala and D.C. Gajdusek, *J*. *Neuroimmunol.,* 5, p. 191-196 (1983); T.S. Elizan, J. Casals and M.D. Yahr, *J*. *Neurol*. *Sci*., 59, p. 341-347 (1983). The autoantibodies against normal CNS neurofilaments react with the paired helical filaments in neurofibrillary tangles characteristic of Alzheimer's Disease. D. Dahl and A. Bignami, *Exp*. *Neurol*., 58, p. 74-80 (1978); M.E. Bruce, *J*. *Neuropathol*. *Exp*. *Neurol*., 37, *p. 595,* abstract (1978).

Animal models for these CNS disorders, which are induced with aluminum chloride or B,B'-iminodipropionitrite (IDPN) to form paired helical filaments in neurofibrillary tangles, also react with antibodies directed against CNS neurofilaments. J.W. Griffen, P.N. Hoffman, A.W. Clark, P.T. Carroll and D.L. Price, *Science,* 202, p. 633-665 (1978).

Control of such autoimmune reactions may lead to the alleviation of symptoms manifested by such reactions. Over the past two decades, a body of clinical literature has accumulated relating to the treatment of autoimmune disease (or, more appropriately, diseases reflecting immune dysfunction) using a technique called provocativeneutralization therapy. Miller, *Annals of Allergy*, 38, p. 185-191 (1977); Miller, *Trans*. *Am*. *Soc*. *Opth*. *& Otolar*. *Allergy*, 14, p. 159-168 (1974); Miller, *Clinical Medicine,* 81, *p.* 16-19 (1974). In short, this method, which is commonly employed for allergy therapy, involves subcutaneous or sublingual introduction of an antigen known, or suspected, to provoke symptoms reflective of immune dysregulation. By serial titration of the provoking material, a concentration of that agent may be determined which will neutralize those symptoms induced by the same substance at a different concentration. That is a prime example of a dose-dependent phenomenon in which one dose induces a positive reaction while another dose of the same agent induces a negative response.

Although it is thought that neutralization occurs as a consequence of reestablishing homeostatic functional levels of T8 suppressor cells, it is quite possible that the same antigen used at a neutralizing concentration to reverse immune dysregulation could also, or instead, trigger endocrine and/or neuronal control mechanisms to reverse symptoms. Because of the intimate association between the three control systems (endocrine, immune, nervous) and proven communication pathways between and among the cells comprising these respective systems, a single active molecule, such as amyloid beta protein in the Alzheimer's victim, and related CNS disorders, may reverse symptoms via any or all of these routes.

Plaque formation is a common component in the etiology of numerous other disease as well. Principal among those are arteriosclerotic diseases. Like Alzheimer's and related diseases, arteriosclerotic diseases, such as atherosclerosis, are plaquing diseases. Such diseases are characterized by arterial plaque formation. These plaques commonly occur in large and medium-sized arteries and generally comprise cells, connective tissue (usually elastin, collagen, and glycosaminoglycans), and lipid deposits. The mixture of those components is usually complex, forming lesions which may be calcified in advanced stages of the disease. Plaque mass slowly increases throughout life, as blood vessels undergo progressive concentric fibromuscular thickening. In atherosclerotic patients, fibromuscular thickening of the intima of blood vessel walls proceeds rapidly and contributes, along with lipid deposition, to restricted blood flow. In non-atherosclerotic patients, the normal thickening of the walls of blood vessels does not contribute to increases in blood pressure and does not compromise blood flow. In fact, plaquing diseases often occur together and patients with neural plaques also have vascular plaques.

It is upon the matrix of fibromuscular thickenings that atherosclerotic plaques develop. Such plaques generally become more prevalent in the third decade of life, with localization being most common in the coronary arteries. Atherosclerotic lesions are generally thought to develop from fatty deposits which transiently occur in all humans in the developed muscular lining of blood vessels. The mechanism of transformation from fatty deposits or "streaks" to atherosclerotic lesions appears to be unknown. However, at least one report suggests that a virus may cause transformation of the normal lipid streaks to atherosclerotic plaques. Melnick, *et al*., *JAMA*, *263:* 2204-207 (1990); wherein it was reported that an avian herpesvirus stimulated atherosclerotic lesions in chickens. The above-cited authors also correlated the presence of cytomegalovirus in humans with atherosclerotic lesions in humans. A finding of herpesvirus and cytomegalovirus antigens, as well as nucleic acids encoding those viruses, in arterial smooth muscle suggests that viral infection of arterial cells may be coincident with the development of atherosclerosis. However, a causative relationship between any virus and atherosclerosis has yet to be conclusively determined.

Also of interest to the present invention is hypertension. The increases in vascular permeability generally observed in hypertension may increase influx of lipoprotein into cells, thus increasing the likelihood of atheroma formation. Hypertension may also contribute to atherosclerosis in blood vessels surrounding the brain. A reduction in hypertension has been shown to significantly reduce the incidence of myocardial infarction associated with atherosclerosis. Other factors in the development and progression of atherosclerosis include diabetes mellitus, which may reduce lipid efflux from cells in the arterial wall. In addition, cigarette smoking dramatically increases the risk of developing atherosclerosis and associated hypertension, including their sequelae, such as infarction of the myocardium and brain. Obesity is another factor which may contribute, especially in an individual who smokes. Overall, hypertension is the single greatest risk factor in coronary diseases as well as cerebrovascular stroke.

The presence of hypertension is a primary indicator of an arteriosclerotic condition and is often used by physicians as the sole diagnostic measure of diseases such as atherosclerosis. Moreover, a reduction of blood pressure is thought to have an effect in reducing the severity of atheroma plaques. The mechanism for such reduction may be a reduction in the transport of lipids and proteins into blood vessels which is coincident with a reduction in blood pressure. The diastolic component of blood pressure is generally thought to be the primary indicator of hypertension. While the systolic component may vary greatly depending upon nervousness, anxiety and the like, diastolic blood pressure generally remains constant and is more reflective of a patient's general vascular state. A diastolic reading of over 90 is considered mild hypertension in an adult and a diastolic reading of over 100 is considered hypertensive and an indicator of arteriosclerotic disease.

### SUMMARY OF THE INVENTION

The present invention provides for alleviating the symptoms of disease states associated with plaque formation. In accordance with the invention, there is provided compositions to stimulate the appropriate metabolic regulatory systems (immune, CNS or endocrine) which retard the progress of the symptoms of plaquing diseases, such as Alzheimer's and related diseases and arteriosclerotic diseases. Observations by scientists have now indicated that the apparent elevated amyloid beta protein concentration in, for example, Alzheimer's diseases may not be due to an increase in genomic expression, but possibly to activation of a mechanism that induces the reorganization of amyloid moieties from normal neurofilaments into paired helical filaments resulting in neurofibrillary tangles, neuritic plaques or amyloid plaques. D. Goldjaber, M.I. Lerman, O.W. McBridge, U. Suffiotti and D.C. Gaidusak, *Science,* 235, p. 77-780 (1987); R.E. Tanzi, J.F. Gusella, P.C. Watkins, G.A.P. Bruns, P. St.George, M.L. Vankeuren, D. Patterson, S. Pagan, D.M. Kurnit and R.L. Neve, *Science,* 235, p. 880-884 (1987); M. Baudry, B.R. Dubrin, L. Beasley, M. Leon and G. Lynch, *Neurobiol*. *Aging,* 7, *p.* 255-260 (1986); G.G. Glenner, *Arch. Path*. *Lab*. *Med*., 107, p. 218-282 (1983). The mechanisms of the present invention may result in triggering control processes that correct the rearrangement of neurofilaments, alter abnormal amyloid protein formation including amyloid beta formation, and/or allow for clearing of axonal transport mechanisms. Similarly, regulatory control systems, as note above, play a role in arteriosclerotic plaque formation, leading to arteriosclerotic diseases such as atherosclerosis. A significant common occurrence in patients having arteriosclerotic disease and/or neural plaquing disease, such as Alzheimer's, is hypertension. Accordingly, compositions of the present invention cause a reduction in hypertension as an indication of alleviation of the overall disease state. The diseases susceptible to treatment with composition according to the invention have in common plaque formation. Accordingly, treatment withcomposition according to the invention provides an effective treatment of all such diseases by alleviating causative symptoms of the disease. In addition, as detailed below, compositions and methods of the invention are useful in the reduction of hypertension generally.

In the invention, a composition is provided comprising amyloid protein and an influenza virus vaccine. In order to identify a dose for use in the invention, a wheal produced upon intradermal injection of the therapeutic material was evaluated according to criteria set forth in Moore, *Clinical Medicine, 81:* 16-19 (1974), incorporated by reference herein. Upon subcutaneous injection, a wheal may be determined to be positive ten minutes after injection as a blanched, hard, raised, and discoid protrusion from the skin. A negative wheal is sufficiently absorbed at the end of ten minutes that the protrusion on the skin has grown less than an average of two millimeters in diameter from its original size.

In a preferred embodiment of the invention, compositions according to the invention comprise a dose from about 10⁻¹⁰ to about 10⁻² mg of amyloid protein and about 0.05 cc of between a 1:5 and 1:125 dilution of an influenza virus vaccine in saline. Thus, the total volume of a typical composition according to the invention for administration to a patient is about 0.05 cc, or one drop. An influenza virus vaccine according to the invention may be any such vaccine, including a commercially-available vaccine, such as Fluogen™ (Parke Davis, Morris Plains, NJ) vaccine. Compositions according to the invention may comprise *β*-amyloid protein or the first 28 amino acids of *β*-amyloid protein.

Also in a preferred embodiment, compositions according to the invention are pharmaceutical compositions for treatment of arteriosclerotic diseases which pharmaceutical compositions comprise amyloid protein and an influenza virus vaccine in a pharmaceutically-acceptable carrier.

Compositions according to the present invention are useful in alleviating symptoms of arteriosclerosis generally, and atherosclerosis in particular. Such methods comprise the step of administering to a patient suspected or confirmed as having an arteriosclerotic disease an effective amount of a pharmaceutical composition comprising amyloid protein and an influenza virus vaccine. An effective amount of a composition according to the invention is an amount which results in a reduction in the symptoms of an arteriosclerotic disease. Most preferably, an effective amount of a composition according to the invention comprises from about 10⁻¹⁰ to about 10⁻² mg of an amyloid protein, preferably β-amyloid protein, and about 0.05 cc of between a 1:5 and 1:125 dilution of an influenza virus vaccine. An amyloid protein used in methods according to the invention may be a *β*-amyloid protein or may be the first 28 amino acids of a *β*-amyloid protein. A highly preferred amount of amyloid protein used in compositions and methods according to the invention is from about 10⁻⁵ and about 10⁻² mg of amyloid protein.

Compositions according to the present invention are effective in alleviating symptoms of any disease in which plaquing is involved and especially diseases in which atheroma formation is characteristic. Compositions and methods according to the invention also alleviate hypertension and reduce cholesterol, both of which have a direct effect in reducing the severity of or eliminating symptoms associated with arteriosclerotic disease. The following detailed description of the invention provides exemplification of claimed methods and compositions. However, it is understood by the skilled artisan that other uses of the invention, specifically relating to the treatment of arteriosclerotic diseases, are within the scope of the present claims.

### A. Application of Materials and Methods to the Treatment of Arteriosclerotic Diseases

Alzheimer's patients treated with amyloid protein as described above show a significant decrease in blood pressure as a result of such treatment which is concomitant with a reduction in symptoms of dementia. As noted above, Melnick, *et al*. report a role for a herpesvirus (Cytomegalovirus, CMV) in atherogenesis. In co-owned, United States Patent No. 4,880,626, it is noted that, while all untreated AIDS patients studied had CMV, none of the patients treated with a fluogen-based composition had CMV. The present application teaches compositions and methods comprising the anti-plaquing amyloid protein and the anti-viral influenza virus vaccine in order to effect treatment of patients presenting with arteriosclerotic conditions. The following examples provide exemplification of the invention through representative embodiments comprising the use of claimed compositions and methods on human subjects. For each example below, original (i.e., pretreatment) blood pressure was taken about three times during each reading to ensure accuracy. Subsequent measures were repeated about 5 times. Unless otherwise noted, patients undergoing treatment according to the invention received 1 drop (sublingual) four times per day. One drop is approximately 0.05 cc of a composition according to the invention.

### EXAMPLE 1

A 54-year-old male patient presented with atherosclerosis, including blood pressure of 140/90. The patient was treated with sublingual drops of a composition comprising 10⁻⁹ mg amyloid protein in a 1:25 dilution of 0.05 cc fluogen™ in saline. The patient was not treated with any other medication during the period in which he was treated with the composition according to the invention. In addition, the patient reported that he remained on a high fat diet and reported no exercise during the treatment period. After daily sublingual treatment (1 drop 4 times per day) for 90 days, the patient's blood pressure had decreased to 117/72. After two years of taking the above composition, the patient's blood pressure has stabilized at about 115/70. The patient's cholesterol also significantly decreased after sustained treatment.

### EXAMPLE 2

A 44-year-old, moderately obese male presented with blood pressure of 140/110 in early August, 1994. The patient was treated with daily sublingual doses (4 times daily) of a composition according to the invention, as recited above in Example 1. The patient received no other medication and did not otherwise alter his lifestyle during the treatment period. By early November, 1993, his blood pressure had decreased to 120/90. In April of 1992, after continued treatment as described above and with no change in lifestyle or diet during the treatment period, the patient had a blood pressure of 123/78. Blood pressure was taken up to five times during each measurement to ensure accuracy.

### EXAMPLE 3

A 55-year old female with initial (pretreatment) blood pressure of about 138/90 began treatment according to methods of the invention and with compositions according to the invention in November, 1992. The patient showed a steady improvement in the diastolic component of blood pressure through three months of treatment. At that point, the patient discontinued treatment and three months later showed increases in diastolic blood pressure. Upon resuming treatment, diastolic blood pressure again decreased. During treatment, the patient was administered compositions as described above in Examples 1 and 2. The following table provides a partial tracking of the patient's blood pressure during treatment and non-treatment periods.

**TABLE 1**

| Treatment | Date | Blood Pressure |
|---|---|---|
| pre-treatment | 11/92 | 138/90 |
| yes | 1/93 | 150/88 |
| yes | 2/93 | 144/82 |
| no | ? | 136/82 |
| no | ? | 118/74 |
| no | ? | 122/80 |
| no | 5/93 | 142/86 |
| yes | 1/94 | 140/80 |
| yes | 4/94 | 130/78 |

### EXAMPLE 4

A 50-year-old male patient presented with blood pressure of 150/104 and began treatment as described above in June, 1992. The results of that treatment are presented in Table 2.

**TABLE 2**

| Treatment | Date | Blood Pressure |
|---|---|---|
| yes | 06/18/93 | 146/95 |
| yes | 06/25/93 | 155/94 |
| yes | 07/02/93 | 138/90 |
| yes | 08/06/93 | 138/78 |
| yes^{*} | 09/93 | 150/98 |
| yes^{*} | 09/93 | 140/96 |
| yes^{*} | 09/93 | 156/108 |
| no | 10/27/93 | 160/102 |
| no | 11/04/93 | 160/100 |
| yes | 04/94 | 130/78 |

| | | |
|---|---|---|
| ^{*}dose of 1-2 drops/day | | |

The results presented in Examples 3 and 4 demonstrate not only the effect of compositions according to the invention in reducing symptoms of arteriosclerosis, but also demonstrate that such symptoms return upon cessation of treatment according to the invention.

### EXAMPLE 5

A 76-year-old female with blood pressure of 210/110 began treatment according to the invention and as described above, using 4 drops per day. After one month of treatment, the patient's blood pressure was reduced to 200/90. After 90 days of treatment her blood pressure was reduced to 160/90.

The foregoing representative results demonstrate that application of compositions according to the invention reduce blood pressure and other symptoms associated with arteriosclerosis. Therefore, treatment compositions according to the invention constitute an effective means for alleviating the symptoms of arteriosclerotic diseases and for completely alleviating such diseases in some cases.

## Claims

1. A pharmaceutical composition for treatment of disease states associated with abnormal accumulation of and/or molecular organisation of amyloid protein or amyloid plaques which comprises amyloid protein or a therapeutically active fragment thereof in an amount effective to alleviate one or more symptoms of said disease state, an effective amount of influenza virus vaccine and a suitable carrier, wherein the disease state is atherosclerosis or hypertension.

2. A pharmaceutical composition according to Claim 1, wherein the amyloid protein is amyloid beta protein.

3. A pharmaceutical composition according to Claim 1, wherein the amyloid protein is a fragment comprising the first 28 amino acid residues of the amyloid beta protein.

4. A pharmaceutical composition according to Claim 1, in the form of a single dosage unit which comprises from about 10⁻¹⁰ to about 10⁻² mg of amyloid beta protein in association with pharmaceutically acceptable excipients.

5. A pharmaceutical composition according to Claim 1, which comprises from 10⁻¹⁰ to 10⁻² mg of amyloid protein and from 0.05 cc of influenza virus vaccine at a dilution of between 1:5 and 1:125.

6. An effective amount of amyloid protein or a therapeutically active fragment thereof in a composition also comprising an influenza virus vaccine for alleviating the symptoms of disease states associated with amyloid plaque formation and/or formation of arterial plaques wherein the disease state is atherosclerosis or hypertension.

7. An effective amount according to Claim 6, wherein the amyloid protein is an amyloid beta protein.

8. An effective amount according to Claim 6, wherein the amyloid protein is a fragment comprising the first 28 amino acid residues of the amyloid beta protein.

9. An effective amount according to Claim 6, 7 or 8, comprising from 10⁻¹⁰ to 10⁻² mg of amyloid protein per dose.

10. An effective amount according to Claim 6, 7 or 8 comprising from 10⁻⁵ to 10⁻³ mg of amyloid protein per dose.

11. An effective amount according to Claim 6, 7 or 8, wherein said composition comprises from 10⁻¹⁰ to 10⁻² mg of amyloid protein and from 0.05 cc of influenza virus vaccine at a dilution of between 1:5 and 1:125.

12. An effective amount according to any one of Claims 6 to 11, wherein said composition is in an amount of about 0.05 cc

13. The use of amyloid protein or a therapeutically active fragment thereof, an effective amount of influenza virus vaccine and a suitable carrier for the manufacture of a medicament for treatment of disease states associated with abnormal accumulation of and/or molecular organisation of amyloid protein or amyloid placques, wherein the disease state is atherosclerosis or hypertension.

## Patentansprüche

1. Eine pharmazeutische Zusammensetzung zur Behandlung von Krankheitszuständen im Zusammenhang mit unnormaler Anreicherung von und/oder molekularer Organisation von Amyloid-Protein oder Amyloid-Plaques, welche Amyloid-Protein oder ein therapeutisch aktives Fragment desselben in einer wirksamen Menge zur Linderung eines oder mehrerer Symptome des besagten Krankheitszustandes, eine wirksame Menge von Influenzavirus-Impfstoff und einen geeigneten Träger umfaßt, wobei der Krankheitszustand Atherosklerose oder Hypertonie ist.

2. Eine pharmazeutische Zusammensetzung nach Anspruch 1, wobei das Amyloid-Protein das Amyloid-Beta-Protein ist.

3. Eine pharmazeutische Zusammensetzung nach Anspruch 1, wobei das Amyloid-Protein ein Fragment, bestehend aus den ersten 28 Aminosäureresten des Amyloid-Beta-Proteins, ist.

4. Eine pharmazeutische Zusammensetzung nach Anspruch 1 in Form einer Einzeldosiseinheit, welche ungefähr 10⁻¹⁰ bis ungefähr 10⁻² mg Amyloid-Beta-Protein zusammen mit pharmazeutisch akzeptablen Arzneistoffträgern umfaßt.

5. Eine pharmazeutische Zusammensetzung nach Anspruch 1, welche von ungefähr 10⁻¹⁰ bis ungefähr 10⁻² mg Amyloid-Protein und 0,05 cm³ Influenzavirus-Impfstoff in einer Verdünnung zwischen 1:5 und 1:125 enthält.

6. Eine wirksame Menge von Amyloid-Protein oder einem therapeutisch aktiven Fragment desselben in einer Zusammensetzung, die auch ein Influenzavirus-Impfstoff umfaßt, zur Linderung der Symptome des Krankheitsstadiums im Zusammenhang mit Amyloid-Plaquebildung und/oder Bildung von arteriellen Plaques, wobei das Krankheitsstadium Atherosklerose oder Hypertonie ist.

7. Eine wirksame Menge nach Anspruch 6, wobei das Amyloid-Protein ein Amyloid-Beta-Protein ist.

8. Eine wirksame Menge nach Anspruch 6, wobei das Amyloid-Protein ein Fragment ist, das die ersten 28 Aminosäurereste des Amyloid-Beta-Proteins umfaßt.

9. Eine wirksame Menge nach Anspruch 6, 7 oder 8, die von 10⁻¹⁰ bis 10⁻² mg Amyloid-Protein pro Dosis enthält.

10. Eine wirksame Menge nach Anspruch 6, 7 oder 8, die von 10⁻⁵ bis 10⁻³ mg Amyloid-Protein pro Dosis enthält.

11. Eine wirksame Menge nach Anspruch 6, 7 oder 8, wobei die genannte Zusammensetzung von 10⁻¹⁰ bis 10⁻² mg Amyloid-Protein und 0,05 cm³ von Influenzavirus-Impfstoff in einer Verdünnung zwischen 1:5 und 1:125 enthält.

12. Eine wirksame Menge nach einem der Ansprüche 6 bis 11, wobei die besagte Zusammensetzung eine Menge von 0,05 cm³ ist.

13. Die Verwendung von Amyloid-Protein oder einem therapeutisch aktiven Fragment desselben, einer wirksamen Menge von Influenzavirus-Impfstoff und einem geeigneten Träger zur Herstellung eines Arzneimittel zur Behandlung von Krankheitssymptomen im Zusammenhang mit unnormaler Anreicherung von und/oder molekularer Organisation von Amyloid-Protein oder Amyloid-Plaques, wobei der Krankheitszustand Atherosklerose oder Hypertonie ist.

## Revendications

1. Composition pharmaceutique pour le traitement d'états maladifs associés à l'accumulation anormale et/ou à l'organisation moléculaire de la protéine amyloïde ou de plaques amyloïdes, qui comprend la protéine amyloïde ou un fragment thérapeutiquement actif de celle-ci dans une quantité efficace pour soulager un ou plusieurs symptômes dudit état maladif, une quantité efficace de vaccin du virus de la grippe, et un support approprié, où l'état maladif est l'athérosclérose ou l'hypertension.

2. Composition pharmaceutique selon la revendication 1, dans laquelle la protéine amyloïde est la protéine amyloïde bêta.

3. Composition pharmaceutique selon la revendication 1, dans laquelle la protéine amyloïde est un fragment comprenant les 28 premiers résidus d'acides aminés de la protéine amyloïde bêta.

4. Composition pharmaceutique selon la revendication 1, sous la forme d'une unité de dosage unique qui comprend d'environ 10⁻¹⁰ à environ 10⁻² mg de la protéine amyloïde bêta en association avec des excipients pharmaceutiquement acceptables.

5. Composition pharmaceutique selon la revendication 1, qui comprend de 10⁻¹⁰ à 10⁻² mg de protéine amyloïde et de 0,05 cm³ de vaccin du virus de la grippe à une dilution d'entre 1 : 5 et 1 : 125.

6. Quantité efficace de protéine amyloïde ou d'un fragment thérapeutiquement actif de celle-ci dans une composition comprenant également un vaccin du virus de la grippe pour soulager les symptômes d'états maladifs associés à la formation de plaques amyloïdes et/ou à la formation de plaques artérielles, où l'état maladif est l'athérosclérose ou l'hypertension.

7. Quantité efficace selon la revendication 6, où la protéine amyloïde est une protéine amyloïde bêta.

8. Quantité efficace selon la revendication 6, où la protéine amyloïde est un fragment comprenant les 28 premiers résidus d'acides aminés de la protéine amyloïde bêta.

9. Quantité efficace selon la revendication 6, 7 ou 8, comprenant de 10⁻¹⁰ à 10⁻² mg de protéine amyloïde par dose.

10. Quantité efficace selon la revendication 6, 7 ou 8, comprenant de 10⁻⁵ à 10⁻³ mg de protéine amyloïde par dose.

11. Quantité efficace selon la revendication 6, 7 ou 8, où ladite composition comprend de 10⁻¹⁰ à 10⁻² mg de protéine amyloïde et de 0,05 cm³ de vaccin du virus de la grippe à une dilution d'entre 1 : 5 et 1 : 125.

12. Quantité efficace selon une quelconque des revendications 6 à 11, où ladite composition est dans une quantité d'environ 0,05 cm³.

13. Utilisation de la protéine amyloïde ou d'un fragment thérapeutiquement actif de celle-ci, d'une quantité efficace de vaccin du virus de la grippe, et d'un support approprié pour la fabrication d'un médicament pour le traitement d'états maladifs associés à l'accumulation anormale et/ou à l'organisation moléculaire de la protéine amyloïde ou de plaques amyloïdes, où l'état maladif est l'athérosclérose ou l'hypertension.
